# EUROPEAN PATENT SPECIFICATION

(11) **EP 2 904 077 B1**
(45) Date of publication and mention of the grant of the patent: **22.08.2018**
(21) Application number: 13773267.3
(22) Date of filing: 07.10.2013
(51) Int. Cl.: C11B 9/00, A61K 8/34, A61K 8/36, A61Q 13/00, A61L 2/18, C07C 33/03, C07C 69/145, C07C 33/02, C07C 33/025, A23L 27/20, A61K 8/37

(54) **FLAVOR AND FRAGRANCE FORMULATION (IV)**
GESCHMACKS- UND DUFTSTOFFFORMULIERUNG (IV)
FORMULATION (IV) D'ARÔME ET DE PARFUM

(30) Priority: 08.10.2012 EP 12187649
(43) Date of publication of application: 12.08.2015
(73) Proprietor: DSM IP Assets B.V., 6411 TE Heerlen (NL)
(72) Inventor: BEUMER, Raphael, CH-4002 Basel (CH); TSCHUMI, Johannes, CH-4002 Basel (CH); GRESSLY, Michael, CH-4002 Basel (CH)
(74) Representative: Kurt, Manfred
(86) International application number: PCT/EP2013/070832
(87) International publication number: WO 2014/056849

(56) References cited:
- CH-A- 284 407
- FR-A- 1 332 794
- GB-A- 777 515
- GB-A- 786 349
- US-A- 2 740 817
- NAVES, YVES-RENÉ; FREI, CHARLES: "Etudes sur les matières végétales volatiles CLXXXVIII. Sur l'absorption d'alcools terpéniques aliphatiques dans l'ultra-violet, de 185 à 225", HELVETICA CHIMICA ACTA, vol. 46, no. 7, 31 December 1963 (1963-12-31), pages 2551-2558, XP002718223, DOI: 10.1002/hlca.19630460711
- BERGER, RALF G. ET AL: "Isolation and Identification of Dry Salami Volatiles", JOURNAL OF FOOD SCIENCE, vol. 55, no. 5, 31 December 1990 (1990-12-31), pages 1239-1242, XP002718224, DOI: 10.1111/j.1365-2621.1990.tb03906.x

## Description

The present invention relates to the use of specific organic compounds as flavor and fragrance material. Furthermore the invention relates to new specific organic compounds, as well as to flavor and fragrance formulations comprising at least one of the specific organic compound.

In the flavor and fragrance industry there is always a need and demand for compounds that enhance, modify, improve or otherwise positively influence an odor note and therefore giving perfumers or other persons the ability to create new fragrances for perfumes, colognes, personal care products, household products or any other products, which comprise flavor and fragrance materials.

Surprisingly it was found that the compounds of formula (I) are very useful as flavor and fragrance material.

Therefore the present invention is related to the use of a compound of formula (I) 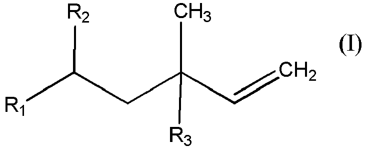 wherein
R₁ signifies -H (hydrogen), or -CH₃ (methyl), and
R₂ signifies a moiety 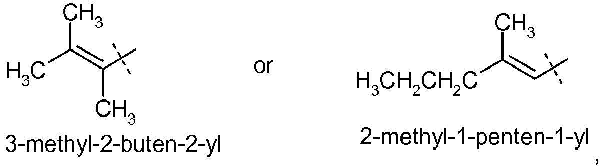
or
R₂ signifies methyl (= "-CH₃"), and
R₃ signifies -OH (hydroxy) or -O(CO)CH₃ (acetyloxy),
   as flavor and fragrance material.

Preferred is the use of at least one compound selected from the group consisting of the compounds of formulae (Ia) - (Ig) and any mixture thereof 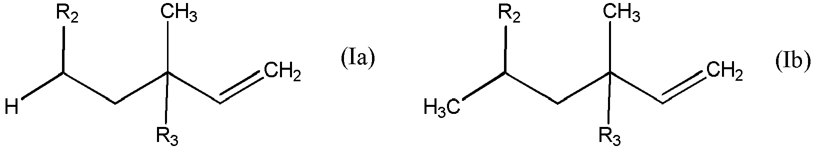 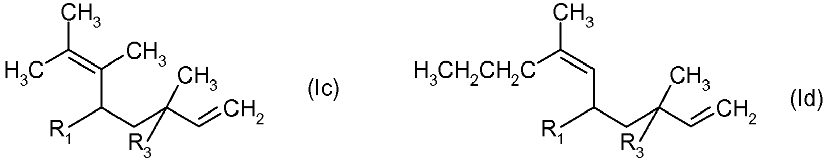 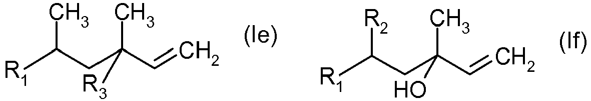 and 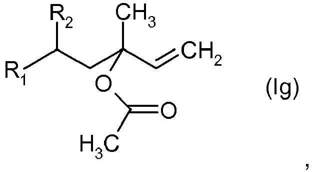 wherein R₁, R₂ and R₃ have the meanings as defined above,
as flavor and fragrance material.

More preferred is the use of at least one compound selected from the group consisting of compounds of formulae (II) - (VII) 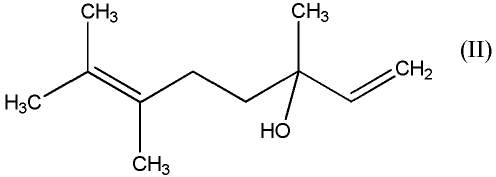 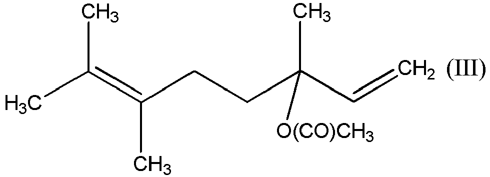 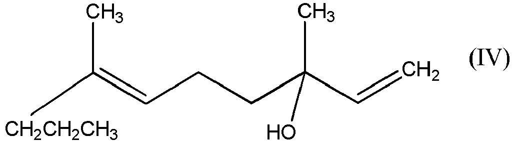 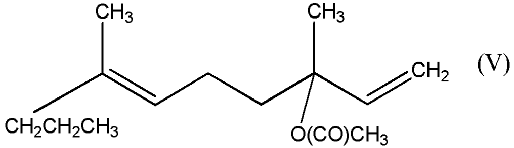 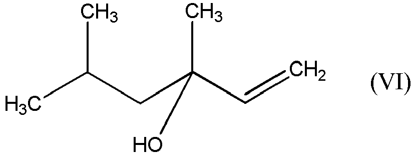 and

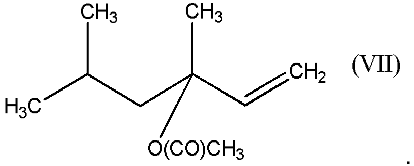

Most preferred is the use of at least one compound selected from the group consisting of compounds of formulae (II), (V), (VI) and (VII).

The compounds of formula (I) may be used as such or in combination with other compounds of formula (I) or other compounds which are known as flavor and fragrance material.

Such other compounds which are known as flavor and fragrance material include all known odorant molecules selected from the extensive range of natural products and synthetic molecules currently available, such as essential oils, alcohols, aldehydes and ketones, ethers and acetals, esters and lactones, macrocycles and heterocycles, and/or in admixture with one or more ingredients or excipients conventionally used in conjunction with odorants in flavor fragrance formulations, for example, carrier materials, and other auxiliary agents commonly used in the art.

The flavor and fragrance material of the present invention is used in a flavor and fragrance formulation.
Such a flavor and fragrance formulation comprises other ingredients.
The flavor and fragrance formulation according to the present invention can be in any form. Usually it is in a solid, gel-like or liquid (or a combination thereof) form. It can also be in an encapsulated form (i.e. a liquid formulation encapsulated by a suitable matrix material).

Therefore the present invention also relates to a perfume, air care product, household product, laundry product, body care product or cosmetic product comprising
(i) at least one compound of formula (I) 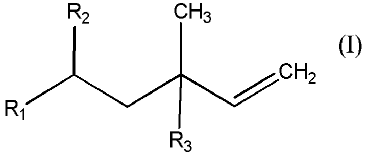
   wherein R₁ signifies - H, or - CH₃, and
   R₂ signifies a moiety 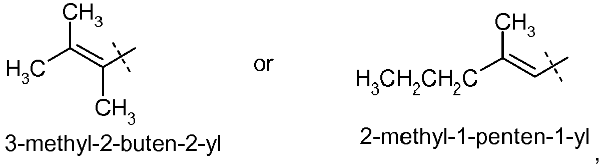
   or
   R₂ signifies - CH₃, and R₃ signifies -OH or -O(CO)CH₃.

Preferred are flavor and fragrance formulations comprising at least one compound selected from the group consisting of the compounds of formulae (Ia) - (Ig) and any mixture thereof 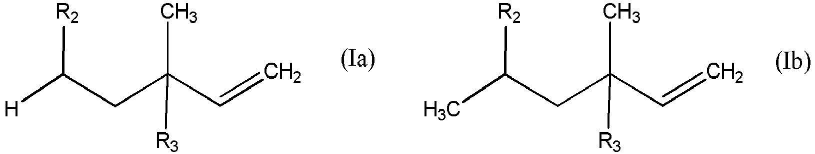 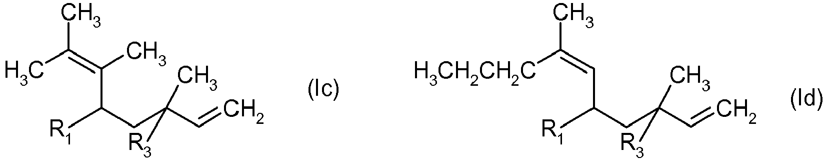 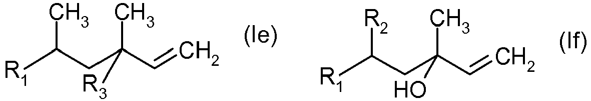 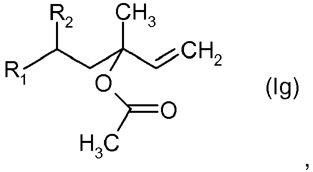 wherein R₁, R₂ and R₃ have the meanings as defined above.

More preferred are flavor and fragrance formulations comprising at least one compound selected from the group consisting of the compounds of formulae (II) - (VII) and any mixture thereof

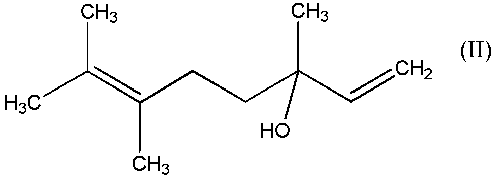

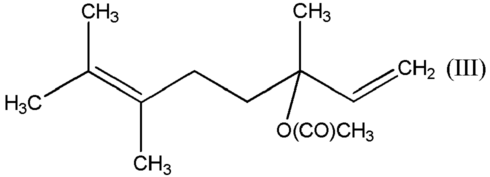

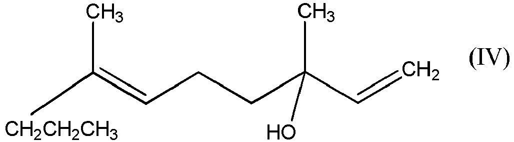

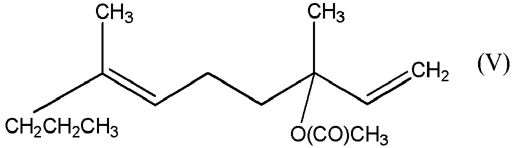

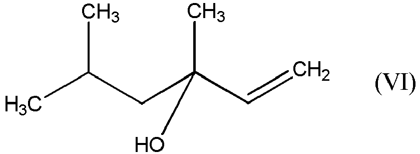

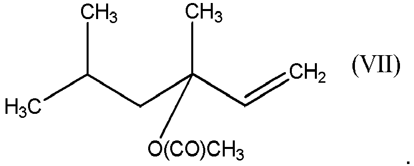

Most preferred are flavor and fragrance formulations comprising at least one compound selected from the group consisting of the compounds of formulae of formulae (II), (V), (VI) and (VII) and any mixture thereof.

When a compound of formula (I) is used in a flavor and fragrance formulation, then the amount thereof is in the range of 0.0001 - 10 weight-% (wt-%), related to the total weight of the flavor and fragrance formulation. Preferred is an amount in the range of 0.01 - 5 wt-%, based on the total weight of the flavor and fragrance formulation.

Therefore the present invention relates to liquid flavor and fragrance formulations comprising
(i) 0.0001 - 10 wt-% (preferably 0.01 - 5 wt-%), related to the total weight of the flavor and fragrance formulation, of at least one compound of formula (I).

Therefore the present invention relates to preferred liquid flavor and fragrance formulations comprising
(i) 0.0001 - 10 wt-% (preferably 0.01 - 5 wt-%), related to the total weight of the flavor and fragrance formulation, of at least one compound chosen from the group consisting of compounds of formulae (Ia) - (Ig).

Therefore the present invention relates to more preferred liquid flavor and fragrance formulations comprising
(i) 0.0001 - 10 wt-% (preferably 0.01 - 5 wt-%), related to the total weight of the flavor and fragrance formulation, of at least one compound chosen from the group consisting of compounds of formulae (II) - (VII) (especially preferred are compounds of formulae (II), (V), (VI) and (VII)).

The flavor and fragrance formulations according to the present invention can comprise further ingredients (= auxiliary compounds), such as any further perfuming compounds solvents, adjuvants, thickeners, surface active agents, pigments, extenders, rheology modifiers, dyestuffs, antioxidants, fillers and the like.

Many flavor and fragrance formulations are in a liquid form (like a perfume, cologne, etc.). Therefore, for such liquid formulation a (diluent) solvent is present. Such common diluents are i.e. dipropyleneglycol, isopropylmyristate, triethylcitrate and alcohols (such as ethanol).

Further examples of fine perfumery are Eau de perfume, Eau de Toilette, Eau de Cologne and Splash Cologne. Fine perfumery products are commonly based on an alcoholic solution as diluent. However fine perfumery products using an oil or wax as diluent are also included within the meaning of this invention. The compounds of formula (I) can be employed in widely varying amounts, depending upon the specific application and on the nature and quantity of other odorant ingredients.
When used in a (fine) perfume, the amount of the compound/s of formula (I) is usually between 0.01 - 10 wt-%, based on the total weight of the (fine) perfume. However, these values and ranges are given only by way of example, since the experienced perfumer may also achieve effects or may create novel accords with lower or higher concentrations.

Furthermore the present invention relates to liquid flavor and fragrance formulations comprising
(i) at least one compound of formula (I), and
(ii) at least one diluent chosen from the group consisting of dipropyleneglycol, isopropylmyristate, triethylcitrate and alcohols (such as ethanol), and optionally
(iii) at least one auxiliary compound selected from the group consisting of perfuming compounds solvents, adjuvants, thickeners, surface active agents, pigments, extenders, rheology modifiers, dyestuffs, antioxidants and fillers.

Furthermore the present invention relates to preferred liquid flavor and fragrance formulations comprising
(i) at least one compound chosen from the group consisting of compounds of formulae (Ia) - (Ig), and
(ii) at least one diluent chosen from the group consisting of dipropyleneglycol, isopropylmyristate, triethylcitrate and alcohols (such as ethanol), and optionally
(iii) at least one auxiliary compound selected from the group consisting of perfuming compounds solvents, adjuvants, thickeners, surface active agents, pigments, extenders, rheology modifiers, dyestuffs, antioxidants and fillers.

Furthermore the present invention relates to more preferred liquid flavor and fragrance formulations comprising
(i) at least one compound chosen from the group consisting of compounds of formulae (II) - (VII) (especially preferred are compounds of formulae (II), (V), (VI) and (VII)), and
(ii) at least one diluent chosen from the group consisting of dipropyleneglycol, isopropylmyristate, triethylcitrate and alcohols (such as ethanol), and optionally
(iii) at least one auxiliary compound selected from the group consisting of perfuming compounds solvents, adjuvants, thickeners, surface active agents, pigments, extenders, rheology modifiers, dyestuffs, antioxidants and fillers.

Furthermore the present invention relates to solid flavor and fragrance formulations comprising
(i) at least one compound of formula (I) and
(ii) at least one auxiliary compound selected from the group consisting of perfuming compounds solvents, adjuvants, thickeners, surface active agents, pigments, extenders, rheology modifiers, dyestuffs, antioxidants and fillers.

Furthermore the present invention relates to preferred solid flavor and fragrance formulations comprising
(i) at least one compound chosen from the group consisting of compounds of formulae (Ia) - (Ig) and
(ii) at least one auxiliary compound selected from the group consisting of perfuming compounds solvents, adjuvants, thickeners, surface active agents, pigments, extenders, rheology modifiers, dyestuffs, antioxidants and fillers.

Furthermore the present invention relates to more preferred solid flavor and fragrance formulations comprising
(i) at least one compound chosen from the group consisting of compounds of formulae (II) - (VII) (especially preferred are compounds of formulae (II), (V), (VI) and (VII)) and
(ii) at least one auxiliary compound selected from the group consisting of perfuming compounds solvents, adjuvants, thickeners, surface active agents, pigments, extenders, rheology modifiers, dyestuffs, antioxidants and fillers.

The compounds of formula (I) may be used in a broad range of flavor and fragrance formulations, e.g. in any field of fine and functional perfumery, such as perfumes, air care products, household products, laundry products, body care products and cosmetics.

The compounds as described hereinabove may be employed in a flavor and fragrance formulation simply by directly mixing at least one compound of formula (I), a mixture thereof, or a fragrance composition with the other ingredients used in the final product, or they may, in an earlier step, be entrapped with an entrapment material, for example, polymers, capsules, microcapsules and nanocapsules, liposomes, film formers, absorbents such as carbon or zeolites, cyclic oligosaccharides and mixtures thereof, or they may be chemically bonded to substrates, which are adapted to release the fragrance molecule upon application of an external stimulus such as light, enzyme, or the like, and then mixed with the other ingredients used in the final product.

Thus, the invention additionally provides a method of manufacturing a flavor and fragrance formulation, comprising the incorporation of a compound of formula (I), as a fragrance ingredient, either by directly admixing the compound to the other ingredients used in the final product or by admixing a fragrance composition comprising a compound of formula (I), which may then be mixed with the other ingredients used in the final product, using conventional techniques and methods.

Thus, the invention additionally provides a preferred method of manufacturing a flavor and fragrance formulation, comprising the incorporation of at least one compound chosen from the group consisting of compounds of formulae (Ia) - (Ig), as a fragrance ingredient, either by directly admixing the compound to the other ingredients used in the final product or by admixing a fragrance composition comprising at least one compound chosen from the group consisting of compounds of formulae (Ia) - (Ig), which may then be mixed with the other ingredients used in the final product, using conventional techniques and methods.

Thus, the invention additionally provides a more preferred method of manufacturing a flavor and fragrance formulation, comprising the incorporation of at least one compound chosen from the group consisting of compounds of formulae (II) - (VII) (especially preferred are compounds of formulae (II), (V), (VI) and (VII)), as a fragrance ingredient, either by directly admixing the compound to the other ingredients used in the final product or by admixing a fragrance composition comprising at least one compound chosen from the group consisting of compounds of formulae (II) - (VII) (especially preferred are compounds of formulae (II), (V), (VI) and (VII)), which may then be mixed with the other ingredients used in the final product, using conventional techniques and methods.

Through the addition of an olfactory acceptable amount of a compound of the present invention as hereinabove described, or a mixture thereof, the odor notes of a consumer product base will be improved, enhanced or modified.

Thus, the invention furthermore provides a method for improving, enhancing or modifying a consumer product (= final product) base by means of the addition thereto of an olfactory acceptable amount of a compound of formula (I), or a mixture thereof.

Thus, the invention furthermore provides a preferred method for improving, enhancing or modifying a consumer product (= final product) base by means of the addition thereto of an olfactory acceptable amount of at least one compound chosen from the group consisting of compounds of formulae (Ia) - (Ig).

Thus, the invention furthermore provides a more preferred method for improving, enhancing or modifying a consumer product (= final product) base by means of the addition thereto of an olfactory acceptable amount of at least one compound chosen from the group consisting of compounds of formulae (II) - (VII) (especially preferred are compounds of formulae (II), (V), (VI) and (VII)).

In the context of the present invention the olfactory effective amount is to be understood as the amount of the at least one compound of formula (I) in a flavor and fragrance formulation will contribute to its particular olfactory characteristics, but the olfactory effect of the flavor and fragrance formulation will be the sum of the effects of each of the perfumes or fragrance ingredients. Thus the compounds of the invention can be used to alter the aroma characteristics of the flavor and fragrance formulation, or by modifying the olfactory reaction contributed by another ingredient in the composition. The amount will vary depending on many factors including other ingredients, their relative amounts and the effect that is desired.

As used herein, "consumer product (=final product)" means a composition for use as a consumer product to fulfill specific actions, such as cleaning, softening, and caring or the like. Examples of such products include fine perfumery, e.g. perfume and eau de toilette; fabric care, household products and personal care products such as laundry care detergents, rinse conditioner, personal cleansing composition, detergent for dishwasher, surface cleaner; laundry products, e.g. softener, bleach, detergent; body care products, e.g. shampoo, shower gel; air care products and cosmetics, e.g. deodorant, vanishing creme. This list of products is given by way of illustration and is not to be regarded as being in any way limiting.

The compounds of formula (I) may be prepared using methods known to the person skilled in the art of organic synthesis.

Furthermore the present invention relates to the following compound of formula (V) which is a novel compound:

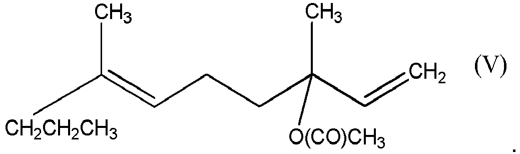

As stated above this compound is produced (manufactured, synthesized) using well known chemical reactions.

A preferred process for the manufacture of compound (V) starts from 6-methyl-5-nonen-2-on which is ethinylated to 3,7-dimethyl-6-decen-1-in-3-ol which is then acylated to 3,7-dimethyl-6-decen-1-in-3-yl acetate. The subsequent hydrogenation of the C=C triple bond to the C=C double bond then leads to the compound of formula (V).

The invention is now further illustrated in the following non-limiting examples.

### Examples

All compounds were evaluated by a panel of four persons for their intensity whereby a range of 1 to 10 was used (1 = very low intensity; 10 = very high intensity). Furthermore these four persons also described the odor of the compounds. The tenancy was evaluated by one person after 3, 6, 8, 24, 48, 72 and 96 hours. For such evaluations a piece of paper was immersed in each single liquid compound as such.

### Example 1: Olfactory properties of the compound of formula II

Odor description: pharmaceutical; fresh herb; green leaves, pleasant.
Intensity: 4.5.
Tenancy: 3-6 hours.

### Example 2: Olfactory properties of the compound of formula III

Odor description: green; herb; flowery; fresh; soil.
Intensity: 6.
Tenancy: 6-8 hours.

### Example 3: Olfactory properties of the compound of formula IV

Odor description: fruity; tropical fruits; sweet; candy.
Intensity: 3.5.
Tenancy: 8-24 hours.

### Example 4: Manufacture of the compound of formula V and its olfactive properties

### a) Manufacture of 3,7-dimethyl-6-decen-1-in-3-ol by ethinylation of 6-methyl-5-nonen-2-on

1360.0 g of 6-methyl-5-nonen-2-on are put in an autoclave under nitrogen and cooled down to a temperature of 15°C. 2548.0 g of ammonia (NH₃) are added. The reaction mixture is cooled again to 15°C. Then acetylene (C₂H₂) is added. The reaction mixture is cooled again to 15°C. Then 26.4 g of a 40 weight-% aqueous potassium hydroxide (KOH) solution are added continuously. After the end of the reaction the reaction mixture is neutralized with acetic acid, extracted with water and the solvent removed. The resulting raw product is then distilled to obtain 3,7-dimethyl-6-decen-1-in-3-ol.

### b) Manufacture of 3,7-dimethyl-6-decen-1-in-3-yl acetate by acylation of 3,7-dimethyl-6-decen-1-in-3-ol

609.0 g of 3,7-dimethyl-6-decen-1-in-3-ol and 0.54 g of p-toluene sulfonic acid in water are mixed and heated up to a temperature of 40°C. 414.1 g of acetic acid anhydride are added within 2 hours. After ca. 20 hours the reaction mixture is cooled down and distilled to obtain 3,7-dimethyl-6-decen-1-in-3-yl acetate.

### c) Manufacture of the compound of formula (V) by hydrogenation of 3,7-dimethyl-6-decen-1-in-3-yl acetate

314.0 g of 3,7-dimethyl-6-decen-1-in-3-yl acetate, 3.0 g of Lindlar catalyst (5% Pd + 3.5% Pb on CaCO₃), 0.01 g of ethylenedithiodiethanol and 0.14 g of zinc acetate are put in an autoclave and heated under nitrogen to a temperature of 40°C. Nitrogen is exchanged by hydrogen (H₂) and the reaction mixture put at an absolute pressure of 2 bar. After the calculated amount of hydrogen has been consumed the reaction mixture is filtered and distilled (2 mbar, 140°C) to obtain 3,7-dimethyl-1,6-decadien-3-yl acetate (= compound of formula V).
Odor description: green chilli pepper; green chili; bamboo.
Intensity: 5.
Tenancy: 8-24 hours.

### Example 5: Olfactory properties of the compound of formula VI

Odor description: tiger balsam; camphor; old cellar; cellar in the countryside.
Intensity: 9.
Tenancy: 3-6 hours.

### Example 6: Olfactory properties of the compound of formula VII

Odor description: freshness; camphor like; menthol; coniferous wood; chrysanthemum, poison for flys.
Intensity: 6.5.
Tenancy: 3-6 hours.

## Claims

1. Use of a compound of formula (I) 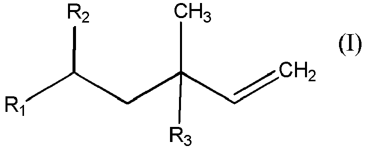 wherein
R₁ signifies -H, or -CH₃, and
R₂ signifies 3-methyl-2-buten-2-yl or 2-methyl-1-penten-1-yl or methyl, and R₃ signifies -OH or -O(CO)CH₃,
as flavor and fragrance material.

2. Use according to claim 1, wherein at least one compound of formulae (Ia) - (Ig) 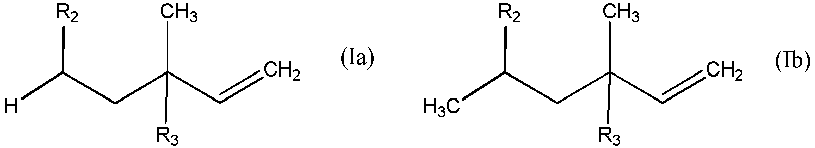 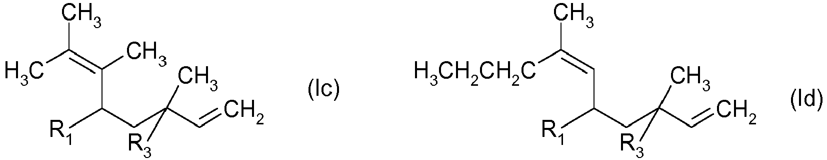 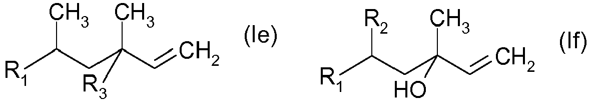 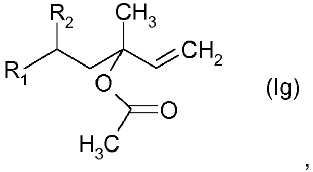 wherein R₁, R₂ and R₃ have the meanings as defined in claim 1,
is used.

3. Use according to any of the preceding claims, wherein at least one compound selected from the group consisting of compounds of formulae (II) - (VII) 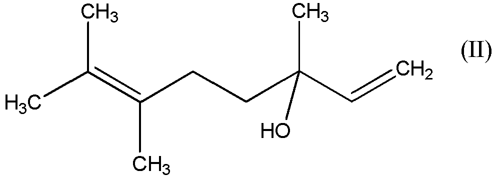 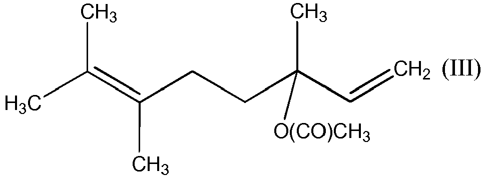 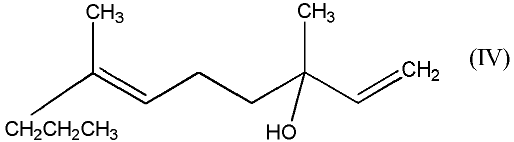 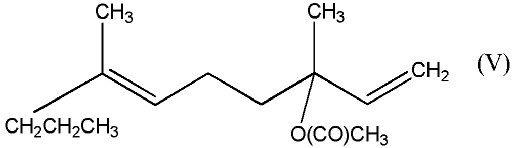 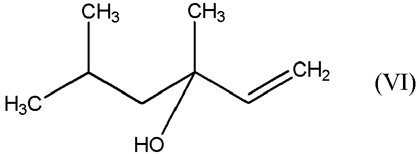 and 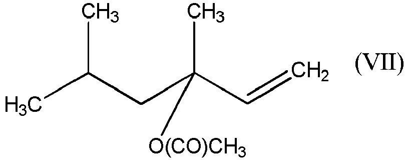 is used.

4. Use according to any of the preceding claims, wherein at least one compound selected from the group consisting of compounds of formulae (II), (V), (VI) and (VII) is used.

5. A perfume, air care product, household product, laundry product, body care product or cosmetic product comprising
(i) at least one compound of formula (I) 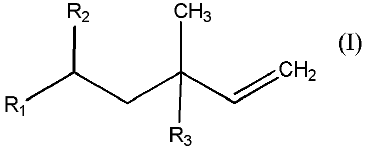 wherein
R₁ signifies -H, or -CH₃, and
R₂ signifies 3-methyl-2-buten-2-yl or 2-methyl-1-penten-1-yl or methyl, and R₃ signifies -OH or -O(CO)CH₃.

6. A flavor and fragrance formulation according to claim 5, comprising at least one compound selected from the group consisting of compounds of formulae (II) - (VII) (preferably compounds of formulae (II), (V), (VI) and (VII)) 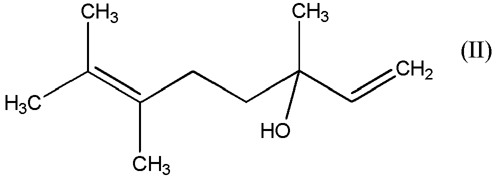 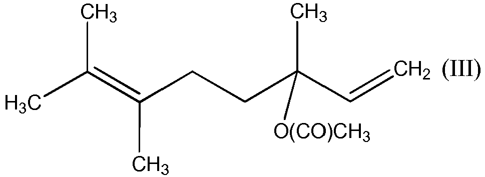 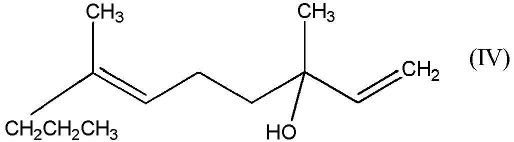 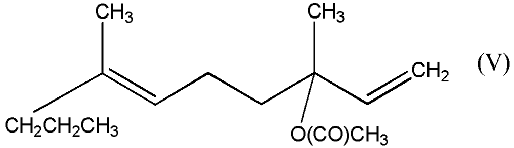 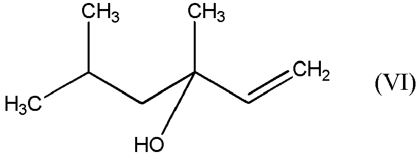 and

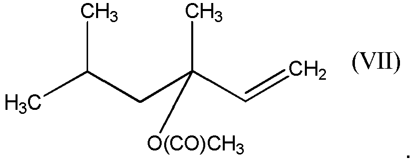

7. Flavor and fragrance formulation according to claim 5 or 6 comprising 0.0001 - 10 wt-%, related to the total weight of the flavor and fragrance formulation, of at least one compound of formula (I).

8. Flavor and fragrance formulation according to claim 5, 6 and/or 7, wherein the flavor and fragrance formulation is solid, gel-like or liquid.

9. A method of improving, enhancing or modifying a flavor and fragrance formulation by means of addition thereto an olfactory acceptable amount of at least one compound of formula (I) 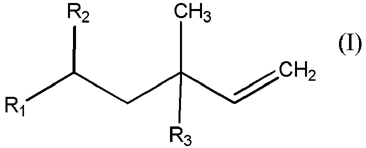 wherein
R₁ signifies -H, or -CH₃, and
R₂ signifies 3-methyl-2-buten-2-yl or 2-methyl-1-penten-1-yl or methyl, and R₃ signifies -OH or -O(CO)CH₃.

10. Method according to claim 9, adding at least one compound selected from the group consisting of compounds of formulae (II) - (VII) (preferably compounds of formulae (II), (V), (VI) and (VII)) 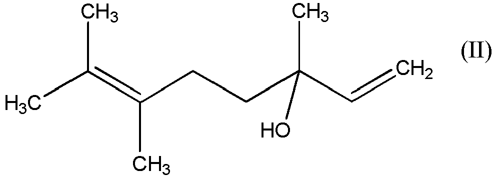 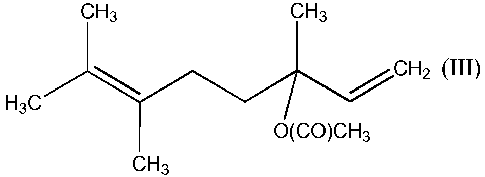 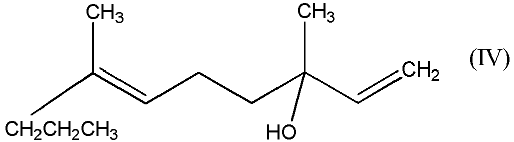 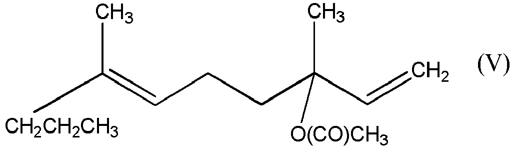 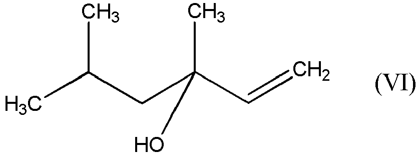 and

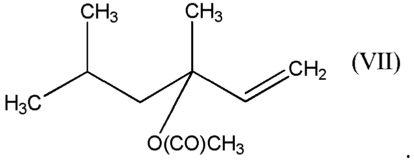

11. Compound of formula (V)

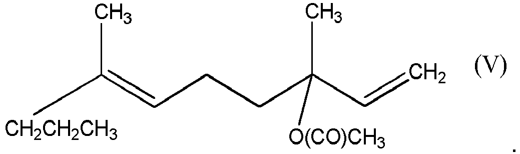

## Patentansprüche

1. Verwendung einer Verbindung der Formel (I) 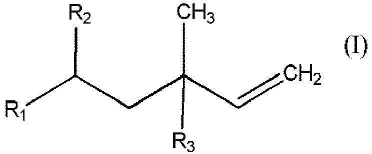 wobei
R₁ für -H oder -CH₃ steht und
R₂ für 3-Methyl-2-buten-2-yl oder 2-Methyl-1-penten-1-yl oder Methyl steht
und R₃ für -OH oder -O(CO)CH₃ steht,
als Geschmacks- und Duftstoff.

2. Verwendung nach Anspruch 1, wobei mindestens eine Verbindung der Formeln (Ia)-(Ig) 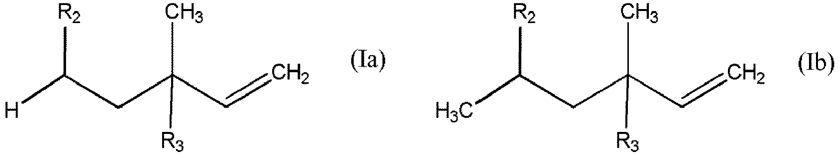 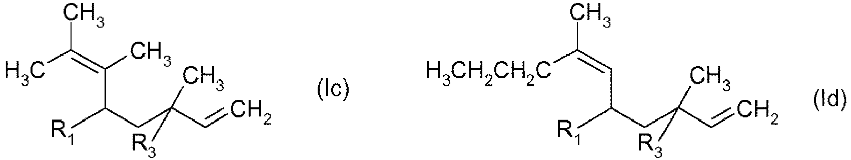 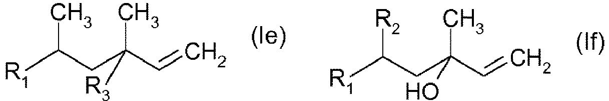 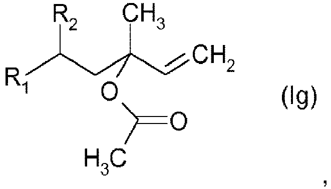 wobei R₁, R₂ und R₃ die in Anspruch 1 angegebenen Bedeutungen haben,
verwendet wird.

3. Verwendung nach einem der vorhergehenden Ansprüche, wobei mindestens eine Verbindung aus der Gruppe bestehend aus Verbindungen der Formeln (II)-(VII) 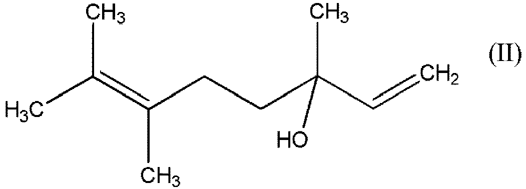 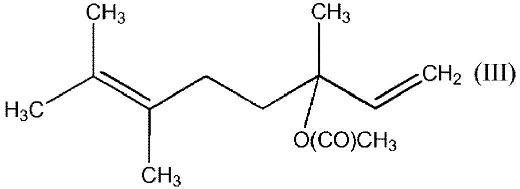 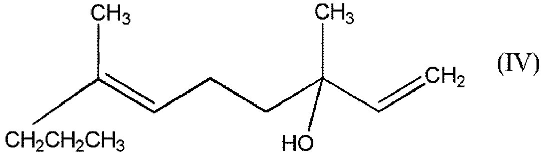 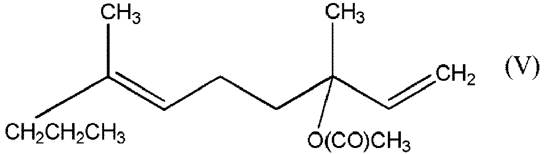 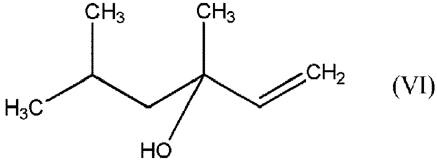 und 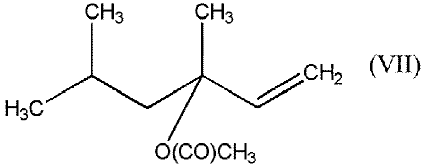 verwendet wird.

4. Verwendung nach einem der vorhergehenden Ansprüche, wobei mindestens eine Verbindung aus der Gruppe bestehend aus Verbindungen der Formeln (II), (V), (VI) und (VII) verwendet wird.

5. Parfüm, Luftpflegeprodukt, Haushaltsprodukt, Waschmittelprodukt, Körperpflegeprodukt oder Kosmetikprodukt, umfassend
(i) mindestens eine Verbindung der Formel (I) 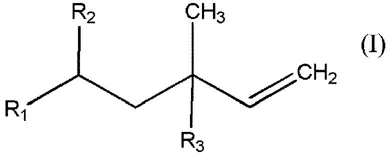 wobei
R₁ für -H oder -CH₃ steht und
R₂ für 3-Methyl-2-buten-2-yl oder 2-Methyl-1-penten-1-yl oder Methyl steht
und R₃ für -OH oder -O(CO)CH₃ steht.

6. Geschmacks- und Duftstoffformulierung nach Anspruch 5, umfassend mindestens eine Verbindung aus der Gruppe bestehend aus Verbindungen der Formeln (II)-(VII) (vorzugsweise Verbindungen der Formeln (II), (V), (VI) und (VII)) 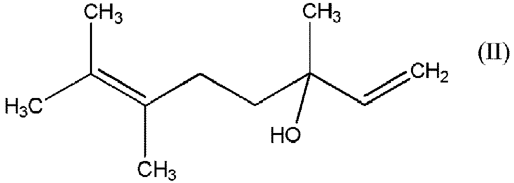 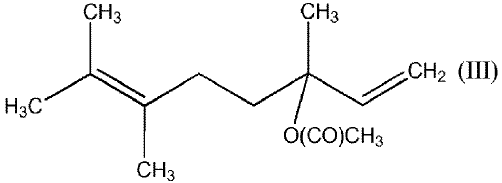 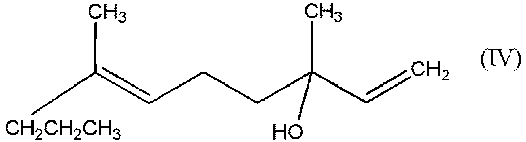 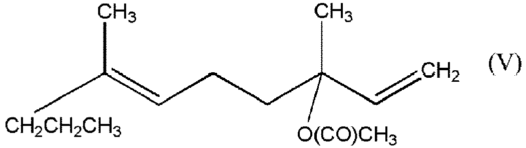 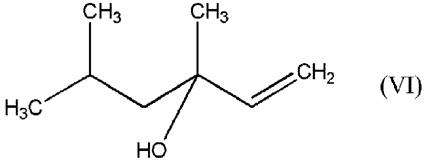 und

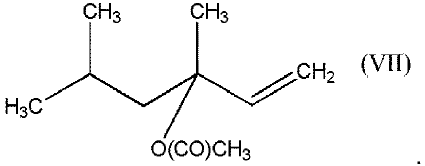

7. Geschmacks- und Duftstoffformulierung nach Anspruch 5 oder 6, umfassend 0,0001-10 Gew.-%, bezogen auf das Gesamtgewicht der Geschmacks- und Duftstoffformulierung, mindestens einer Verbindung der Formel (I).

8. Geschmacks- und Duftstoffformulierung nach Anspruch 5, 6 und/oder 7, wobei die Geschmacks- und Duftstoffformulierung fest, gelartig oder flüssig ist.

9. Verfahren zur Verbesserung, Verstärkung oder Modifizierung einer Geschmacks- und Duftstoffformulierung durch Zugeben einer olfaktorisch unbedenklichen Menge mindestens einer Verbindung der Formel (I) 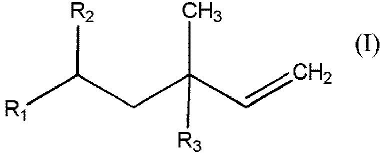 wobei
R₁ für -H oder -CH₃ steht und
R₂ für 3-Methyl-2-buten-2-yl oder 2-Methyl-1-penten-1-yl oder Methyl steht
und R₃ für -OH oder -O(CO)CH₃ steht.

10. Verfahren nach Anspruch 9, bei dem mindestens eine Verbindung aus der Gruppe bestehend aus Verbindungen der Formeln (II)-(VII) (vorzugsweise Verbindungen der Formeln (II), (V), (VI) und (VII)) 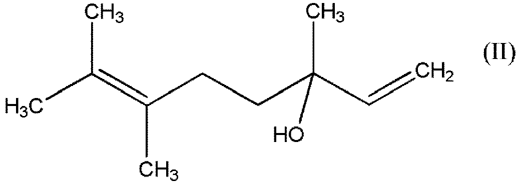 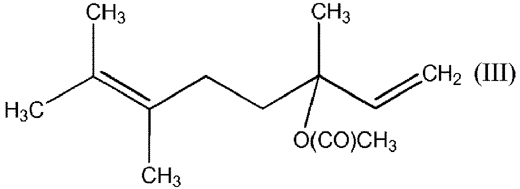 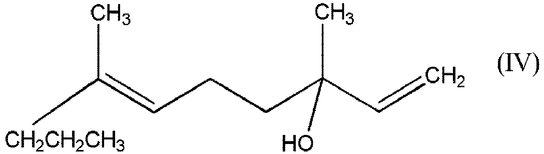 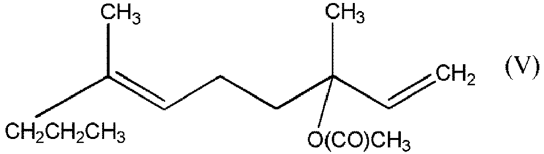 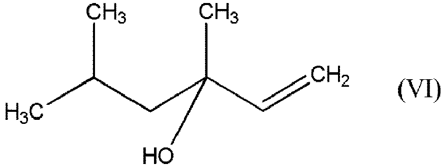 and 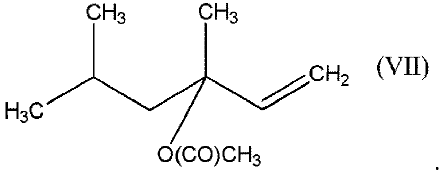 zugegeben wird.

11. Verbindung der Formel (V)

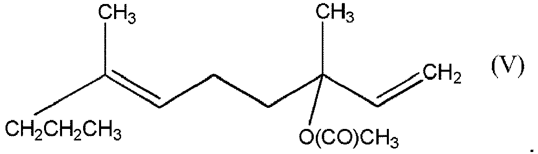

## Revendications

1. Utilisation d'un composé de formule (I) 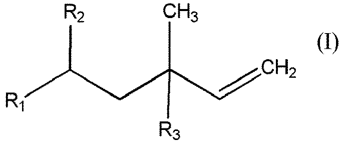 où
R₁ désigne -H ou -CH₃ et
R₂ désigne un groupe 3-méthylbut-2-én-2-yle ou 2-méthylpent-1-én-1-yle ou méthyle et
R₃ désigne -OH ou -O(CO)CH₃,
en tant que substance d'arôme et de parfum.

2. Utilisation selon la revendication 1, dans laquelle au moins un composé représenté par les formules (Ia) - (Ig) 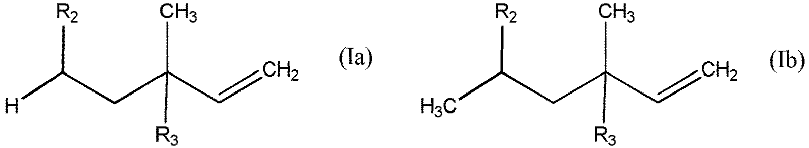 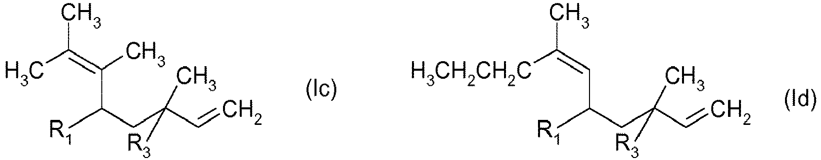 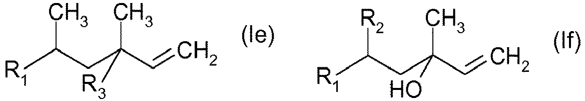 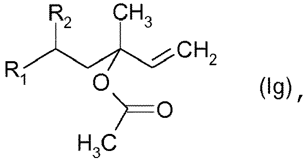 où R₁, R₂ et R₃ ont les significations telles que définies dans la revendication 1,
est utilisé.

3. Utilisation selon l'une quelconque des revendications précédentes, dans laquelle au moins un composé choisi dans le groupe constitué par les composés représentés par les formules (II)-(VII) 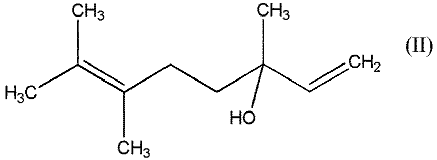 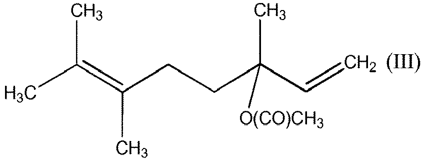 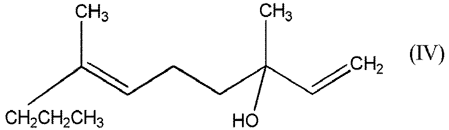 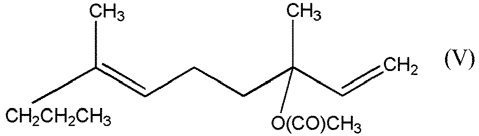 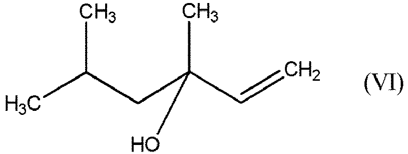 et 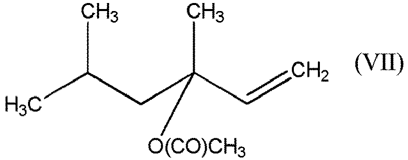 est utilisé.

4. Utilisation selon l'une quelconque des revendications précédentes, dans laquelle au moins un composé choisi dans le groupe constitué par les composés représentés par les formules (II), (V), (VI) et (VII) est utilisé.

5. Parfum, produit de traitement de l'air, produit ménager, produit pour la lessive, produit de soins corporels ou produit cosmétique comprenant
(i) au moins un composé de formule (I) 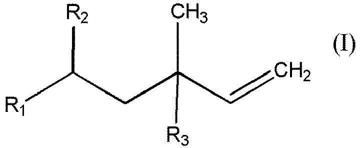 où
R₁ désigne -H ou -CH₃ et
R₂ désigne un groupe 3-méthylbut-2-én-2-yle ou 2-méthylpent-1-én-1-yle ou méthyle et
R₃ désigne -OH ou -O(CO)CH₃.

6. Formulation d'arôme et de parfum selon la revendication 5, comprenant au moins un composé choisi dans le groupe constitué par les composés représentés par les formules (II)-(VII) (de préférence les composés représentés par les formules (II), (V), (VI) et (VII)) 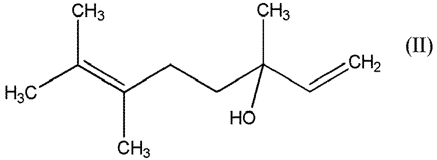 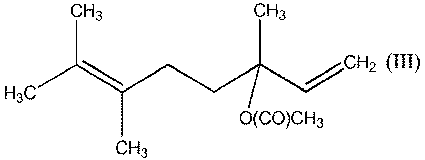 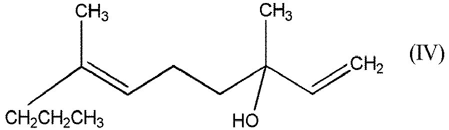 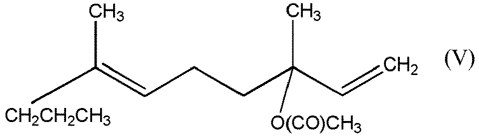 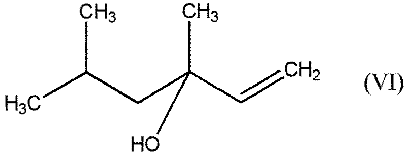 et

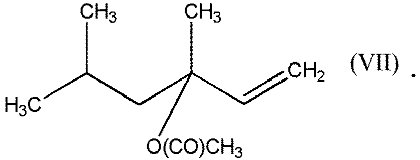

7. Formulation d'arôme et de parfum selon la revendication 5 ou 6 comprenant 0,0001-10 % en poids, par rapport au poids total de la formulation d'arôme et de parfum, d'au moins un composé de formule (I).

8. Formulation d'arôme et de parfum selon la revendication 5, 6 et/ou 7, la formulation d'arôme et de parfum étant solide, sous forme de gel ou liquide.

9. Procédé d'amélioration, d'amplification ou de modification d'une formulation d'arôme et de parfum au moyen de l'ajout à celle-ci d'une quantité olfactivement acceptable d'au moins un composé de formule (I) 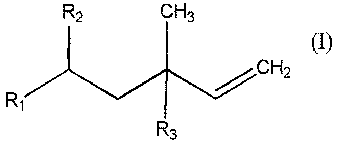 où
R₁ désigne -H ou -CH₃ et
R₂ désigne un groupe 3-méthylbut-2-én-2-yle ou 2-méthylpent-1-én-1-yle ou méthyle et
R₃ désigne -OH ou -O(CO)CH₃.

10. Procédé selon la revendication 9, ajoutant au moins un composé choisi dans le groupe constitué par les composés représentés par les formules (II)-(VII) (de préférence les composés représentés par les formules (II), (V), (VI) et (VII)) 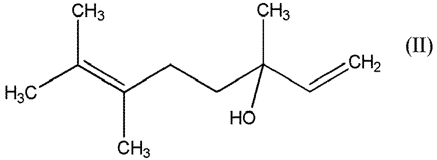 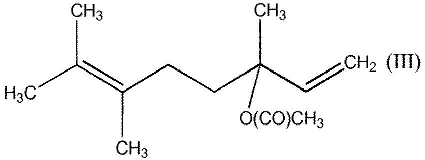 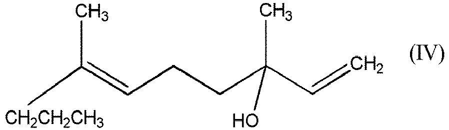 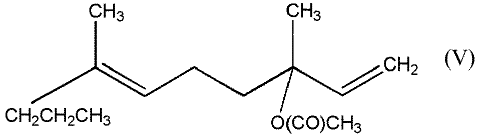 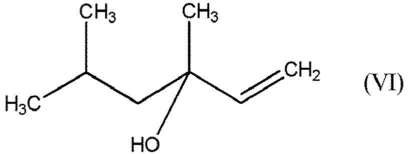 et

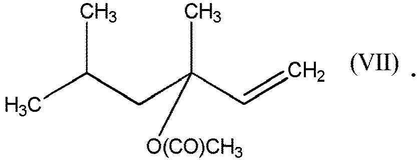

11. Composé de formule (V)
